# EUROPEAN PATENT APPLICATION

(11) **EP 4 356 931 A1**
(43) Date of publication of application: **24.04.2024**
(21) Application number: 22824232.7
(22) Date of filing: 15.06.2022
(51) Int. Cl.: A61K 48/00, C12N 1/00, C12N 15/86

(54) **PRPF31 VARIANT AND USE THEREOF**

(30) Priority: 16.06.2021 CN 202110665266
(71) Applicant: Chigenovo Co., Ltd., Beijing 102206 (CN)
(72) Inventor: ZHONG, Xiancheng, Beijing 102206 (CN); CHEN, Shaohong, Beijing 102206 (CN); ZHANG, Fan, Beijing 102206 (CN)
(74) Representative: Hoffmann Eitle
(86) International application number: PCT/CN2022/098879
(87) International publication number: WO 2022/262756

(57) **Abstract**

The present application provides a PRPF31 variant comprising an exogenous nuclear localization (NLS) domain, wherein the exogenous NLS domain comprises an amino acid sequence set forth in any of SEQ ID NOs. 3 to 4. The PRPF31 variant can improve the biological activity of a retinal pigment epithelium (RPE) cell, and can treat a disease caused by PRPF31 gene mutation.

## Description

### TECHNICAL FIELD

The present application relates to the field of biomedicine, and specifically to a PRPF31 variant and uses thereof.

### BACKGROUND

Retinitis pigmentosa (RP) is a type of progressive retinal degenerative disease caused by the degenerations of retinal photoreceptors and retinal pigment epitheliums. It is an inherited blindness-causing eye disease clinically characterized by night blindness, progressive visual field narrowing and vision loss, retinal pigmentation, sallow and atrophied optic disc, and abnormal electroretinogram. RP involves various modes of inheritance, including autosomal dominant inheritance (ADRP).

The RHO gene encodes the rhodopsin protein, which is the main protein involved in the visual transduction pathway. Approximately 30-40% of ADRP patients have RHO gene mutations. PRPF31 gene is the abbreviation for the pre-mRNA processing factor 31 gene, which is involved in the pre-mRNA splicing process. Among Chinese ADRP patients, about 8-10% are caused by PRPF31 gene mutations.

Therefore, it is currently urgent to restore the normal gene splicing in RP cells and thereby restore cell functions.

### SUMMARY

The present application provides a PRPF31 variant and uses thereof, wherein the PRPF31 variant comprises an exogenous nuclear localization (NLS) domain with a specific amino acid sequence. The PRPF31 variant described herein also involves the following properties: (1) localization in the nucleus; (2) promoting the mRNA splicing of the gene; (3) improving the phagocytic function of the induced RPE cell; (4) increasing the cilium length of the induced RPE cell; and (5) treating a disease caused by PRPF31 gene mutation(s). The present application further provides use of the PRPF31 variant in the manufacture of a medicament for treating a disease caused by PRPF31 gene mutation(s), use in improving the biological activity of the induced RPE cell, and/or use in improving the mRNA splicing efficiency.

In one aspect, the present application provides a PRPF31 variant comprising an exogenous nuclear localization (NLS) domain or truncated version thereof, wherein the exogenous NLS domain comprises an amino acid sequence set forth in any of SEQ ID NOs. 3 to 4.

In certain embodiments, the PRPF31 variant does not comprise the NLS domain of wild-type PRPF31.

In certain embodiments, the PRPF31 variant comprises an NOSIC domain.

In certain embodiments, the NOSIC domain comprises an amino acid sequence set forth in SEQ ID NO. 35.

In certain embodiments, the PRPF31 variant comprises an NOP domain.

In certain embodiments, the NOP domain comprises an amino acid sequence set forth in SEQ ID NO. 36.

In certain embodiments, the PRPF31 variant is capable of splicing the mRNA of a gene, wherein the gene comprises the RHO gene.

In certain embodiments, the PRPF31 variant is derived from human PRPF31.

In certain embodiments, the PRPF31 variant comprises an amino acid sequence set forth in any of SEQ ID NOs. 20 to 21.

In another aspect, the present application provides a nucleic acid molecule encoding the PRPF31 variant described herein.

In certain embodiments, the nucleic acid molecule comprises a nucleotide sequence set forth in any of SEQ ID NOs. 28 to 29.

In another aspect, the present application provides a vector comprising the nucleic acid molecule described herein.

In certain embodiments, the vector comprises a viral vector.

In certain embodiments, the vector comprises an AAV vector.

In certain embodiments, the vector comprises a lentiviral vector.

In certain embodiments, the serotype of the AAV vector comprises AAV1, AAV2, AAV5, AAV6, AAV8, AAV9, and/or AAV10.

In certain embodiments, the lentivirus comprises human immunodeficiency virus (HIV), simian immunodeficiency virus (SIV), and non-primate lentiviruses such as feline immunodeficiency virus (FIV), equine infectious anemia virus (EIAV), bovine immunodeficiency virus (BIV), and/or Visna-Maedi virus (VMV).

In another aspect, the present application provides the use of the PRPF31 variant described herein, the nucleic acid molecule described herein, and/or the vector described herein in the manufacture of a medicament for treating a disease.

In certain embodiments, the disease comprises a disease caused by PRPF31 gene mutation(s).

In certain embodiments, the disease comprises retinitis pigmentosa (RP).

In certain embodiments, the disease comprises autosomal dominant retinitis pigmentosa (ADRP).

In another aspect, the present application provides a method of promoting the biological activity of a retinal pigment epithelium (RPE) cell, comprising the step of administrating to the RPE cell the PRPF31 variant described herein, the nucleic acid molecule described herein, and/or the vector described herein.

In certain embodiments, the biological activity comprises the phagocytic activity of the RPE cell, and/or the cilium length of the PRE cell.

In certain embodiments, the induced RPE cell is derived from a pluripotent stem cell and/or a totipotent stem cell.

In certain embodiments, the induced RPE cell is derived from a human iPS cell.

In certain embodiments, the PRPF31 variant does not comprise the NLS domain of wild-type PRPF31.

In certain embodiments, the PRPF31 variant comprises an amino acid sequence set forth in any of SEQ ID NOs. 20 to 21.

In certain embodiments, the administrating comprises administrating a vector comprising a nucleic acid molecule encoding the PRPF31 variant.

In certain embodiments, the nucleic acid molecule comprises a nucleotide sequence set forth in any of SEQ ID NOs: 28 to 29.

In certain embodiments, the vector comprises a viral vector.

In another aspect, the present application provides a method of improving mRNA splicing efficiency, comprising the step of administrating to a cell containing the mRNA a PRPF31 variant, wherein the PRPF31 variant comprises an exogenous nuclear localization (NLS) domain, and the exogenous NLS domain comprises an amino acid sequence set forth in any of SEQ ID NOs. 3 to 4.

In certain embodiments, the PRPF31 variant does not comprise the NLS domain of wild-type PRPF31.

In certain embodiments, the PRPF31 variant comprises an amino acid sequence set forth in any of SEQ ID NOs. 20 to 21.

In certain embodiments, the administrating comprises administrating a vector comprising a nucleic acid molecule encoding the PRPF31 variant.

In certain embodiments, the nucleic acid molecule comprises a nucleotide sequence set forth in any of SEQ ID NOs: 28 to 29.

In certain embodiments, the vector comprises a viral vector.

Other aspects and advantages of the present application can be readily appreciated by those skilled in the art from the detailed descriptions below. Only exemplary embodiments of the present application are shown and described in the detailed descriptions below. As will be recognized by those skilled in the art, the content of the present application will enable those skilled in the art to make changes to the particular embodiments without departing from the spirit and scope of the invention disclosed in the present application. Accordingly, the accompanying drawings and the descriptions in the specification of the present application are only exemplary and not restrictive.

### DESCRIPTION OF THE DRAWINGS

The specific features of the invention disclosed in the present application are set forth in the appended claims. The characteristics and advantages of the invention disclosed in the present application can be better understood by reference to the exemplary embodiments described in detail below and the accompanying drawings. Brief descriptions of the accompanying drawings are as follows:
Fig. 1 shows the main distribution results of detecting the expression of human PRPF31 in cells by using fluorescent labeling.
Fig. 2 shows the main distribution results of detecting the expression of human PRPF31 in cells by using fluorescent labeling and DAPI staining.
Fig. 3 shows the effect of human PRPF31 on the mRNA splicing of RHO reporter gene.
Fig. 4 shows the verification results of cells transfected with human PRPF31.
Fig. 5 shows the main distribution results of detecting the expression of the PRPF31 variant described herein in cells by using fluorescent labeling.
Fig. 6 shows the main distribution results of detecting the expression of the PRPF31 variant described herein in cells by using fluorescent labeling.
Fig. 7 shows the verification results of cells transfected with the PRPF31 variant described herein.
Fig. 8 shows the effect of the PRPF31 variant described herein on the mRNA splicing of RHO reporter gene.
Fig. 9 shows the efficiency of the PRPF31 variant described herein for the mRNA splicing of RHO reporter gene.
Fig. 10 shows the effect of the PRPF31 variant described herein on the phagocytic function of human RPE cells.
Fig. 11 shows the effect of the PRPF31 variant described herein on the phagocytic function of human RPE cells.
Fig. 12 shows the effect of the PRPF31 variant described herein on the phagocytic function of human RPE cells.
Fig. 13 shows the effect of the PRPF31 variant described herein on the cilium length of human RPE cells.
Fig. 14 shows the effects of the PRPF31 variant described herein on the phagocytic function and the cilium length of human IPSC-induced RPE cells.

### DESCRIPTION OF EMBODIMENTS

The embodiments of the invention in the present application are described below by certain specific Examples, and those skilled in the art can easily understand other advantages and effects of the invention in the present application from the disclosure of this specification.

### Definitions of terms

As used herein, the term "PRPF31" generally refers to a pre-mRNA processing factor, which is a component of spliceosome U4/U6.U5 trisnRNP (small nuclear ribonucleoprotein) subunit. PRPF31 can be responsible for the splicing of pre-mRNA in the nucleus. The PRPF31 gene has a full length of 16 kb, contains 14 exons, and is located on chromosome 19q13.4. The protein encoded by PRPF31 has a full length of 499 amino acids and a molecular weight of 61 kD. PRPF31 can contain three domains: NOSIC domain, NOP domain, and NLS domain. The NOSIC domain and the NOP domain can be considered as RNA-binding domains, while the NLS domain can be responsible for localizing the translated PRPF31 in the cytoplasm to the nucleus so as to complete its pre-mRNA splicing function. Among them, the NOSIC domain and the NOP domain are relatively conserved. The NOSIC domain can be the amino acids at positions 93-144 in the human PRPF31; and the NOP domain can be the amino acids at positions 190-334 in the human PRPF31. The absence of PRPF31 could lead to abnormal mRNA splicing in cells, while the effects on photoreceptor cells and retinal pigment epithelium (RPE) cells are especially serious.

As used herein, the term "variant" generally refers to a polypeptide or protein that differs from polypeptides or proteins derived therefrom by one or more length changes or sequence changes. The polypeptide or protein from which the polypeptide variant or protein variant is derived may also be referred to as the parent polypeptide or protein (e.g., PRPF31 protein). The term "variant" may comprise "a fragment(s)" or "a derivative(s)" of the parent polypeptide or protein. Generally, the "fragment" may be smaller in length or size than the parent molecule, and the derivative may exhibit one or more differences in sequence from the parent molecule. As used herein, the variant may also encompass modified molecules such as, but not limited to, post-translationally modified proteins (e.g., glycosylated, biotinylated, phosphorylated, ubiquitinated, palmitoylated, or proteolytically cleaved proteins). The variant may be artificially constructed, for example constructed by genetic technology, while the parent polypeptide or protein may be a wild-type polypeptide or protein. However, a naturally occurring variant should also be understood as being encompassed by the "variant" described herein. In addition, the variant described herein may also be derived from homologs, orthologs, or paralogs of the parent molecule, or derived from artificially constructed variants, provided that the variant exhibits at least one biological activity (i.e., functional activity) of the parent molecule. As used herein, the variant may have a certain degree of sequence identity to the parent polypeptide or parent protein from which it is derived. For example, the variant may have at least 80% sequence identity to the parent polypeptide thereof. For example, the identity may be at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%.

As used herein, the term "nuclear localization domain (NLS)" generally refers to an amino acid sequence that facilitates the entry of a protein into the nucleus, for example via nuclear transport. The nuclear localization domain may be a domain or domains capable of mediating the nuclear import, or the retention in the nucleus, of a protein or polynucleotide. The nuclear localization domain may be known to those skilled in the art. For example, the NLS sequence can be found in WO/2001/038547. The representative examples of NLS include, but are not limited to, single-component nuclear localization signals, two-component nuclear localization signals, as well as N-terminal and C-terminal motifs. The N-terminal basic domain generally conforms to the consensus sequence K-K/R-X-K/R, which was originally discovered in the SV40 large T antigen and represents a single-component NLS. A non-limiting example of the N-terminal basic domain NLS is PKKKRKV. The two-component nuclear localization signals are also known, which contain two clusters of basic amino acids spaced by about 10 amino acids. The N-terminal and C-terminal motifs include, for example, the acidic M9 domain of hnRNP Al, and the complex signal of the sequence KIPIK and U snRNP in the yeast transcriptional repressor Matα2. Most of these NLS can be directly recognized by specific receptors of the importin β family.

As used herein, the term "RHO" generally refers to Rhodopsin 2, Opsin-2, Opsin 2, OPN2, CSNBAD1, or RP4. The rhodopsin is located in the rods outer segment (ROS), and is necessary for normal vision, especially for the perception of weak light stimuli. Rods are a kind of retinal photoreceptor cells responsible for scotopic vision. Cones are another kind of retinal photoreceptor cells in retina that are responsible for photopic vision and color vision. At the ROS, the rhodopsin usually binds to 11-*cis* retinal (11cRAL) which is a derivative form of vitamin A. The absorption of photons by ROS turns the rhodopsin into active rhodopsin (R*), and isomerizes 11cRAL to all-trans retinal (atRAL). The atRAL is quickly reduced to all-trans retinol (atROL) after separation from R*. The interphotoreceptor retinoid-binding protein (IRBP) is responsible for transferring the atROL into RPE cells, where the atROL is converted by the lecithin retinol acyltransferase (LRAT) to all-trans retinol ester, which is further converted to 11-cis retinol ester and then isomerized to 11-cis retinol (11cROL) by the hydrolytic isomerase RPE65. The 11cROL is oxidized by RDHs to 11cRAL, which binds to IRBP and is transported back to photoreceptor cells for reuse. R* converts the GDP on the α subunit of transducin G (Gt) in the downstream membranous disc into GTP, such that the α subunit is separated from the βγ subunits, the cyclic guanosine monophosphate-phosphodiesterase 6 (cGMP-PDE6) is activated, and the cGMP is hydrolyzed. The concentration of cellular cGMP is reduced to close the cGMP-gated cation channel of OS, such that the Ca²⁺ concentration within photoreceptor cells is reduced, the cell membrane is hyperpolarized, and the light signal is converted into a visual electrical signal. After the phototransduction is terminated, the photoreceptor cells are subjected to a series of chemical reactions and return to the non-illuminated state. At this time, R* is phosphorylated and binds to the arrestin to inhibit the downstream signaling pathway. The PDE6 is in an inactive state, while the cGMP is synthesized in the rods. The increased cGMP concentration makes the cation channel open to achieve Ca²⁺ influx, and the cell membrane is depolarized. Such nerve impulses triggered by the opening and closing of cGMP-gated cation channels are transmitted to the visual center of the cerebral cortex through the connections between the synaptic terminals of photoreceptor cells and the neurons at all levels in the retina as well as the optic nerve to form the vision.

The human *RHO* gene is located at position 22.1 of the long arm of chromosome 3 (3q22.1), and the molecule is from 129,528,639 bp to 129,535,344 bp in chromosome 3 (*Homo sapiens,* Annotation release, Version 109.20200228, GRCh38.p13, NCBI). The nucleotide sequence of the *RHO* gene can be found in NCBI GenBank Accession No. NG_009115.1. The *RHO* gene has 5 exons. Table 1 shows the exon identifiers of the *RHO* gene and the exon initiation/termination sites in the Ensembl database.

**Table 1. Exons of the RHO gene**

| Exon | Exon ID | Initiation/termination site (chromosome 3) |
|---|---|---|
| 1 | ENSE00001079597 | 129,528,639-129,529,094 |
| 2 | ENSE00001152211 | 129,530,876-129,531,044 |
| 3 | ENSE00001152205 | 129,532,251-129,532,416 |
| 4 | ENSE00001152199 | 129,532,533-129,532,772 |
| 5 | ENSE00001079599 | 129,533,608-129,535,344 |

As used herein, the term "nucleic acid molecule" generally refers to a polymeric form of nucleotides (such as deoxyribonucleotides or ribonucleotides) of any length, or analogs thereof. The polynucleotide can have any three-dimensional structure and can perform any known or unknown function. The non-limiting examples of the polynucleotide are as follows: coding or non-coding region of gene or gene fragment, multiple loci (one locus) defined by ligation analysis, exon, intron, messenger RNA (mRNA), transporter RNA, ribosomal RNA, short interfering RNA (siRNA), short hairpin RNA (shRNA), micro-RNA (miRNA), ribozyme, cDNA, recombinant polynucleotide, branched polynucleotide, plasmid, vector, isolated DNA of any sequence, isolated RNA of any sequence, nucleic acid probe, and primer. The polynucleotide may contain one or more modified nucleotides, such as methylated nucleotides and nucleotide analogs. If present, the modification of nucleotide structure can be performed before or after polymer assembly. The sequence of the nucleotide can be interrupted by a non-nucleotide component. The polynucleotide can be further modified after polymerization, such as by conjugation to a labeling component.

As used herein, the term "vector" generally refers to a nucleic acid delivery vehicle into which a polynucleotide encoding a protein can be inserted to express the protein. Through the transformation, transduction, or transfection of a host cell with the vector, the genetic elements carried by the vector could be expressed in the host cell. For example, the vector comprises: plasmid; phagemid; cosmid; artificial chromosome, such as yeast artificial chromosome (YAC), bacterial artificial chromosome (BAC), or P1-derived artificial chromosome (PAC); phage, such as λ phage or M13 phage; viral vector; and the like. A vector may contain a variety of elements controlling expressions, comprising promoter sequence, transcription initiation sequence, enhancer sequence, selection element, and reporter gene. Additionally, the vector may also contain a replication origin. The vector may also comprise a component contributing to the entry into a cell, such as viral particle, liposome, or protein coat, but not limited to these substances.

As used herein, the term "viral vector" generally refers to a non-wild-type recombinant viral particle serving as a gene delivery vehicle and containing a recombinant viral genome packaged inside a viral capsid. The animal virus species used as the vector may include retrovirus (including lentivirus), adenovirus, adeno-associated virus (AAV), herpesvirus (such as herpes simplex virus), poxvirus, baculovirus, papillomavirus, and papovavirus (such as SV40).

As used herein, the term "AAV" is the standard abbreviation for adeno-associated virus. The adeno-associated virus is a single-stranded DNA parvovirus that grows only in cells, some functions of which are provided by the co-infection of helper virus. Currently, there are thirteen AAV serotypes that have been characterized, as shown in Table 1 below. General information and reviews on AAV can be found, for example, in Carter, 1989, Handbook of Parvoviruses, Vol. 1, pp. 169-228, and Berns, 1990, Virology, pp. 1743-1764, Raven Press, (New York). However, it is fully expected that these identical principles will apply to additional AAV serotypes, since the various serotypes are known to be very closely related, both structurally and functionally, even at the genetic level. For example, all AAV serotypes apparently exhibit very similar replication properties mediated by homologous rep genes; and all carry three related capsid proteins, such as those expressed in AAV6. The degree of correlation is further demonstrated by heteroduplex analysis, revealing the extensive cross-hybridization along the length of the genome between serotypes; as well as the presence of similar self-annealing segments at the end of inverted terminal repeat (ITR). Similar infection patterns also suggest that the replication functions in each serotype are under similar regulatory control.

**Table 2. AAV capsid protein serotype**

| AAV serotype | NCBI GenBank accession number |
|---|---|
| AAV1 | NC_002077.1 |
| AAV2 | NC_001401.2 |
| AAV3 | NC_001729.1 |
| AAV3B | AF028705.1 |
| AAV4 | NC_001829.1 |
| AAV5 | NC_006152.1 |
| AAV6 | AF028704.1 |
| AAV7 | NC_006260.1 |
| AAV8 | NC_006261.1 |
| AAV9 | AX753250.1 |
| AAV10 | AY631965.1 |
| AAV11 | AY631966.1 |
| AAV12 | DQ813647.1 |
| AAV13 | EU285562.1 |

As used herein, the term "AAV vector" generally refers to a vector comprising one or more polynucleotides (or transgenes) of interest flanked by AAV inverted terminal repeats (ITRs). Such AAV vectors can be replicated and packaged into infectious virus particles when present in host cells that have been transfected with vectors encoding and expressing the rep and cap gene products. The term "AAV virion" or "AAV virus particle" or "AAV vector particle" refers to a virus particle composed of at least one AAV capsid protein and an encapsidated polynucleotide AAV vector. If the particle contains a heterologous polynucleotide (i.e., a polynucleotide other than the wild-type AAV genome, such as a transgene to be delivered into mammalian cells), it is often referred to as an "AAV vector particle" or simply referred to as "AAV vector." Thus, the production of AAV vector particles necessarily includes the production of AAV vectors such that the vectors are contained within the AAV vector particles.

The AAV "rep" gene and "cap" gene refer to the genes encoding the replication protein and capsid protein, respectively. The AAV rep and cap genes have been found in all AAV serotypes studied to date and are described herein and in the references cited. In the wild-type AAV, the rep and cap genes are generally adjacent to each other in the virus genome (i.e., they are "coupled" together into contiguous or overlapping transcriptional units), and they are generally conserved across AAV serotypes. The AAV rep and cap genes may also be referred to individually or collectively as "AAV packaging genes." The AAV cap gene encodes a Cap protein capable of packaging the AAV vector in the presence of rep and adenovirus helper functions and capable of binding to target cell receptors. In certain instances, the AAV cap gene encodes a capsid protein derived from a particular AAV serotype, such as those shown in Table 1.

The different serotypes of AAV have genomic sequences that are significantly homologous at the amino acid and nucleic acid levels, provide a set of similar genetic functions, produce virions that are physically and functionally substantially equivalent, and are replicated and assembled through nearly identical mechanisms.

As used herein, the term "retinitis pigmentosa (RP)" generally refers to a class of inherited blindness-causing fundus oculi diseases caused by abnormal functions of retinal photoreceptors, mostly single-gene genetic diseases. The main inheritance modes of RP include autosomal dominant (ADRP), autosomal recessive (ARRP), and X-linked inheritances. RP11 is an ADRP caused by a genetic mutation in PRPF31, and the probability of RP11 in ADRP is about 10%.

As used herein, the term "retinal pigment epithelium (RPE) cell" generally refers to a layer of pigment cells immediately outside the retinal sensory nerves. The retinal pigment epithelium consists of a single layer of hexagonal cells that contain dense pigment granules. The retinal pigment epithelium (RPE) is closely connected with the underlying choroid and the upper retinal nerve cells. Its main functions may include: controlling the fluids and nutritions in the subretinal space; functioning as a blood-retinal barrier; synthesizing the growth factor for adjusting the local structure; absorbing lights and regulating the electrical balance; regenerating and synthesizing visual pigments; phagocytosing and digesting photoreceptor outer segments; maintaining retinal attachment; and regenerating and repairing after injury. RPE is generally considered to be an important tissue for maintaining the photoreceptor function, and is also affected by many lesions in the choroid and retina.

As used herein, the term "retinal pigment epithelium (RPE) atrophy" generally refers to degenerative changes manifested by cell death or dysfunction in the retinal pigment epithelium (RPE). The age-related macular degeneration or retinitis pigmentosa (RP) is often accompanied by the retinal pigment epithelium atrophy. The retinitis pigmentosa (abbreviated as RP), also known as the retinal pigment lesion, usually refers to a class of inherited ocular diseases. There are three modes of inheritance: autosomal recessive, dominant, and X-linked recessive, and dihybrid inheritance and mitochondrial inheritance are also present. The common symptoms in the early stage may be night blindness, narrowing of visual field, the ability to see the scene right ahead but the inability to see the visual field slightly to the left or right, and then the gradual disappearance of vision. RP may include uniocular primary retinitis pigmentosa, sector primary retinitis pigmentosa, central or paracentral primary retinitis pigmentosa, retinitis pigmentosa sine pigmento, albescent punctate degeneration of retina, Bietti's crystalline dystrophy, pigmented paravenous retinitis pigmentosa, preserved para-arteriolar retinal pigment epithelium retinitis pigmentosa, Leber congenital amaurosis, and retinitis pigmentosa in other syndromes.

As used herein, the term "pharmaceutical composition" generally refers to a composition suitable for administration to a patient such as human patient. For example, the pharmaceutical composition described herein may comprise the nucleic acid molecule described herein, the vector described herein, and/or the cell described herein, and optionally a pharmaceutically acceptable adjuvant. In addition, the pharmaceutical composition may also comprise one or more (pharmaceutically effective) vehicles, stabilizers, excipients, diluents, solubilizers, surfactants, emulsifiers, and/or preservatives for suitable formulations. The acceptable ingredients of the composition are not toxic to recipients at the dosages and concentrations employed. The pharmaceutical composition of the present application includes, but is not limited to, liquid, frozen, and lyophilized compositions.

As used herein, the terms "polypeptide," "peptide," and "protein" are used interchangeably, and generally refer to a polymer of amino acids of any length. The polymer may be linear or branched, and it may contain modified amino acids, and may be interrupted by non-amino acids. These terms also encompass amino acid polymers that have been modified. These modifications may include: disulfide bond formation, glycosylation, lipidation, acetylation, phosphorylation, or any other manipulation (e.g., binding to a labeling component). The term "amino acid" includes natural and/or non-natural or synthetic amino acids, including glycine and D and L optical isomers, as well as amino acid analogs and peptidomimetics.

As used herein, the term "pluripotent stem cell" generally refers to a stem cell having the ability to form the cell and tissue ultimately found in an entire organism, but incapable of forming an entire organism. For example, the pluripotent stem cell can proliferate extendedly or virtually indefinitely *in vitro* while maintaining the undifferentiated state thereof and exhibiting a normal karyotype (chromosome). The pluripotent stem cell may have the ability to differentiate into all three germ layers (ectoderm, mesoderm, and endoderm) under appropriate conditions. For example, the pluripotent stem cell may comprise ES cells isolated from early embryos and/or analogous EG cells isolated from fetal-stage primordial germ cells.

As used herein, the term "totipotent stem cell" generally refers to a stem cell having a complete differentiation versatility, that is, a stem cell having the ability to grow into multiple cell types of any fetal or adult mammalian body. For example, the totipotent stem cell may have the potential to differentiate into three germ layers (ectoderm, mesoderm, and endoderm), thus giving rise to any fetal or adult cell type. The totipotent stem cell may comprise embryonic stem (ES) cells, embryonic germ (EG) cells, and embryonic carcinoma (EC) cells.

As used herein, the term "and/or" should be understood to mean either or both of the options.

As used herein, the term "comprise" or "include" generally means the inclusion of expressly specified features, but without the exclusion of other elements.

As used herein, the term "about" generally refers to variations above or below the specified value within the range of 0.5%-10%, such as variations above or below the specified value within the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10%.

### DETAILED DESCRIPTION OF THE INVENTION

In one aspect, the present application provides a PRPF31 variant comprising an exogenous nuclear localization (NLS) domain, wherein the exogenous NLS domain comprises an amino acid sequence set forth in any of SEQ ID NOs. 3 to 4.

In the present application, the PRPF31 variant may not comprise the NLS domain of wild-type PRPF31. For example, the PRPF31 variant may not comprise the complete NLS domain of wild-type PRPF31. In some cases, the PRPF31 variant may not have the biological function of the NLS domain of wild-type PRPF31 (such as the function of exogenous nuclear localization). In some cases, in the PRPF31 variant, the NLS domain of wild-type PRPF31, or at least a portion thereof, may be replaced with the exogenous NLS domain. For example, the PRPFR31 variant may comprise the complete exogenous NLS domain.

In the present application, the PRPF31 variant may comprise an NOSIC domain. For example, the NOSIC domain may comprise the amino acids at positions 93-144 in the amino acid sequence of the PRPF31 variant. For example, the NOSIC domain may comprise an amino acid sequence set forth in SEQ ID NO. 35.

In the present application, the PRPF31 variant may comprise an NOP domain. For example, the NOP domain may comprise the amino acids at positions 190-334 in the amino acid sequence of the PRPF31 variant. For example, the NOP domain may comprise an amino acid sequence set forth in SEQ ID NO. 36.

For example, the PRPF31 variant may comprise the exogenous NLS domain, the NOSIC domain, and the NOP domain.

In the present application, the PRPF31 variant is capable of splicing the mRNA of a gene, wherein the gene may comprise RHO gene.

In the present application, the PRPF31 variant can splice the mRNA of a gene. For example, the PRPF31 variant may be a pre-mRNA processing factor gene, and can be involved in the mRNA splicing. The PRPF31 variant may affect the pre-mRNA splicing process of the RHO gene. The PRPF31 variant may affect retinitis pigmentosa (RP), along with the RHP gene. In the present application, the RHO gene may comprise a wild-type RHO gene. The RHO gene may comprise any mutated RHO gene. For example, the RHO minigene reporter gene may comprise a nucleotide sequence set forth in SEQ ID NO. 33.

In the present application, the PRPF31 variant is derived from human PRPF31. For example, the PRPF31 variant may be a PRPF31 variant comprising the exogenous NLS domain based on human PRPF31.

In the present application, the PRPF31 variant comprises an amino acid sequence set forth in any of SEQ ID NOs. 20 to 21.

In the present application, the nucleic acid molecule may encode the exogenous nuclear localization (NLS) domain described herein, wherein the exogenous NLS domain comprises an amino acid sequence set forth in any of SEQ ID NOs. 3 to 4.

For example, the nucleic acid molecule may comprise a nucleotide sequence set forth in any of SEQ ID NOs. 10 to 11.

In another aspect, the present application provides a nucleic acid molecule encoding the PRPF31 variant described herein.

In the present application, the nucleic acid molecule may comprise a nucleotide sequence set forth in any of SEQ ID NOs. 28 to 29.

In another aspect, the present application provides a vector comprising the nucleic acid molecule described herein.

In the present application, the vector may comprise a plasmid, for example a linear plasmid.

In the present application, the vector may comprise a viral vector. In the present application, the viral vector may comprise lentiviral vectors, retroviral vectors, adenoviral vectors, adenovirus-associated virus vectors, and/or herpes simplex virus vectors.

For example, the lentiviral vector may comprise human immunodeficiency virus (HIV), simian immunodeficiency virus (SIV), and non-primate lentiviruses such as feline immunodeficiency virus (FIV), equine infectious anemia virus (EIAV), bovine immunodeficiency virus (BIV), and/or Visna-Maedi virus (VMV).

In the present application, the vector may comprise an AAV vector.

In the present application, the serotype of the AAV vector may comprise AAV1, AAV2, AAV5, AAV6, AAV8, AAV9, and/or AAV10.

In another aspect, the present application provides the use of the PRPF31 variant described herein, the nucleic acid molecule described herein, and/or the vector described herein in the manufacture of a medicament for treating a disease.

The present application provides the PRPF31 variant described herein, the nucleic acid molecule described herein, and/or the vector described herein, for treating a disease.

The present application provides a method of treating a disease, comprising the step of administrating the PRPF31 variant described herein, the nucleic acid molecule described herein, and/or the vector described herein.

In the present application, the disease may comprise a disease caused by PRPF31 gene mutation(s). In some cases, the disease may be associated with functional defects in the retinal pigment epitheliums (RPE). In some cases, the disease may be associated with functional defects in retinal photoreceptor cells.

In the present application, the disease may comprise retinitis pigmentosa (RP). In the present application, the disease may comprise inherited retinal degeneration (IRD).

In the present application, the disease may comprise autosomal dominant retinitis pigmentosa (ADRP).

In another aspect, the present application provides a method of improving the biological activity of a retinal pigment epithelium (RPE) cell, comprising the step of administrating to the RPE cell the PRPF31 variant described herein, the nucleic acid molecule described herein, and/or the vector described herein.

In the present application, the biological activity may comprise the phagocytic activity of the RPE cell, and/or the cilium length of the PRE cell. In the present application, the biological activity may comprise maintaining the selective transport of nutrients and metabolites, expressing and secreting various growth factors, participating in the visual cycle, maintaining the blood-retinal barrier, and/or phagocytizing the outer segment disc membrane detached from the photoreceptor cell.

In the present application, the phagocytic activity may comprise phagocytizing the disc membrane detached from the outer segment of the photoreceptor. The phagocytic activity may also comprise non-specific phagocytosis. For example, the phagocytic activity may comprise phagocytizing the substances without specific identification sites, such as polyethylene microspheres, melanin droplets, or trypan blue dyes.

In the present application, the cilium of the RPE cell may be a primary cilium on the RPE cell (RPE, which may be a small tubular protrusion on the RPE cell). The cilium of the RPE cell may participate in the optical transduction cascade.

In the present application, the induced RPE cell may be derived from a pluripotent stem cell and/or a totipotent stem cell.

In the present application, the pluripotent stem cell may comprise a stem cell having the ability to form the cell and tissue ultimately found in an entire organism, but incapable of forming an entire organism. For example, the pluripotent stem cell can proliferate extendedly or virtually indefinitely *in vitro* while maintaining the undifferentiated state thereof and exhibiting a normal karyotype (chromosome). The pluripotent stem cell may have the ability to differentiate into all three germ layers (ectoderm, mesoderm, and endoderm) under appropriate conditions. For example, the pluripotent stem cell may comprise a mesenchymal stem cell. For example, the pluripotent stem cell may comprise an induced pluripotent stem cell, i.e., iPS cell.

In the present application, the totipotent stem cell may comprise a cell capable of forming all cells and tissues found in an entire organism. For example, the totipotent stem cell can form an entire organism.

For example, the induced RPE cell can be derived from a human iPS cell.

In the present application, the PRPF31 variant may not comprise the NLS domain of wild-type PRPF31.

In the present application, the PRPF31 variant may comprise an amino acid sequence set forth in any of SEQ ID NOs. 20 to 21.

In the present application, the administrating may comprise administrating a vector comprising a nucleic acid molecule encoding the PRPF31 variant.

In the present application, the nucleic acid molecule may comprise a nucleotide sequence set forth in any of SEQ ID NOs. 28 to 29.

In the present application, the vector may comprise a viral vector.

In another aspect, the present application provides a method of improving the mRNA splicing efficiency, comprising the step of administrating to a cell containing the mRNA a PRPF31 variant, wherein the PRPF31 variant comprises an exogenous nuclear localization (NLS) domain, and the exogenous NLS domain comprises an amino acid sequence set forth in any of SEQ ID NOs. 20 to 21.

In the present application, the PRPF31 variant may not comprise the NLS domain of wild-type PRPF31.

In the present application, the PRPF31 variant may comprise an amino acid sequence set forth in any of SEQ ID NOs. 20 to 21.

In the present application, the administrating may comprise administrating a vector comprising a nucleic acid molecule encoding the PRPF31 variant.

In the present application, the nucleic acid molecule may comprise a nucleotide sequence set forth in any of SEQ ID NOs. 28 to 29.

In the present application, the vector may comprise a viral vector.

Without intention to be limited by any theory, the following Examples are only intended to illustrate the PRPF31 variants, preparation methods, uses, etc. in the present application, and are not intended to limit the scope of the claimed invention.

### Examples

### Example 1. Mcherry-PRPF31 was localized in the nucleus

In order to detect the PRPF31 localization, the human PRPF31 cDNA (SQE ID NO. 25) was cloned into the vector pMcherry-N1 (purchased from Clontech) to obtain the vector pMcherry-PRPF31. The vector pMcherry-N1 could express mcherry (a red fluorescent protein useful as a tracer). The nucleotide sequence of the nucleic acid molecule encoding mcherry was set forth in SEQ ID NO. 34.

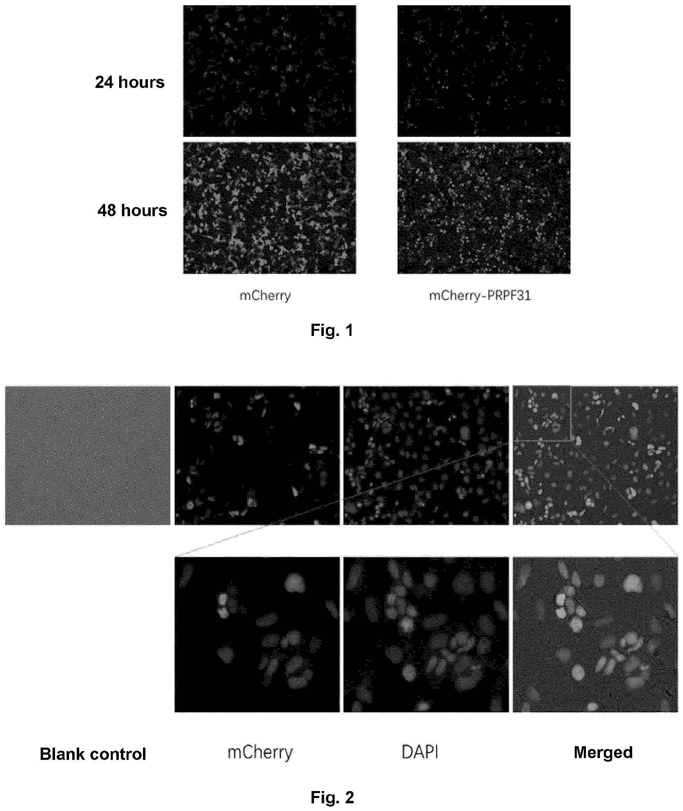

The vector pMcherry-PRPF31 (i.e., mcherry-PRPF31) and the control vector pMcherry-N1 (i.e., mcherry) were transfected into HEK293 cells, respectively, and the red fluorescence expressions were observed at 24 hours and 48 hours after transfection, respectively. The results were shown in Fig. 1.

The cells were observed the red fluorescence expression locations for mCherry at 48 hours after transfection, in addition with DAPI staining. The results in Fig. 1 and Fig. 2 showed that the red fluorescent proteins expressed by the vector pMcherry-PRPF31 were mainly expressed in the nucleus and had a relatively good co-localization with DAPI; while the red fluorescent proteins expressed by the control vector pMcherry-N1 were expressed throughout the cell (including the nucleus and cytoplasm).

### Example 2. Mcherry-PRPF31 could promote the mRNA splicing of RHO reporter gene

In order to detect the pre-mRNA splicing function of PRPF31, the minigene reporter gene for RHO was constructed (the nucleotide sequence set forth in SEQ ID NO. 33).

The region from Exon 3 to Exon 4 of the RHO gene was cloned from the HEK293 cell genome. If this reporter gene was spliced normally, then the intron between E3 and E4 would be excised, resulting in a 229 bp mRNA fragment. On the contrary, if the normal splicing of this reporter gene was inhibited, then the intron between E3 and E4 would be retained, resulting in a 345 bp mRNA fragment. The function of PRPF31 could be characterized by the ratio of 345 bp fragment to 225 bp fragment.

The RHO minigene reporter gene was cloned into the vector pcDNA4 (purchased from Thermo Fisher) to obtain the vector p4-RHO.

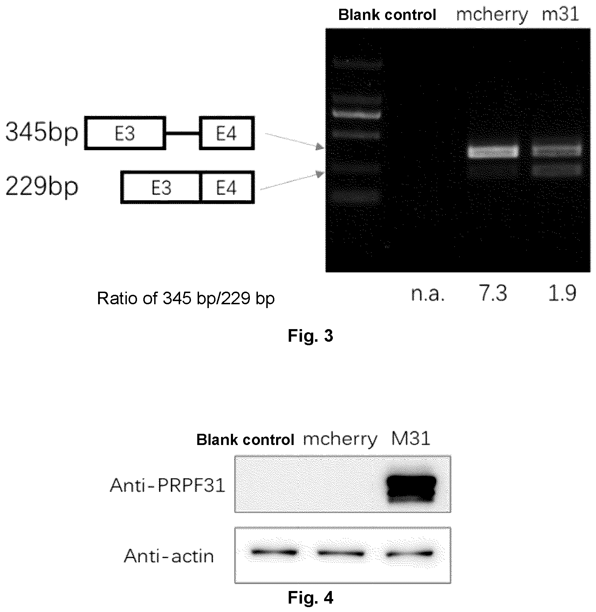

The results were shown in Fig. 3. In the case of co-transfection with the vector p4-RHO and the control vector pMcherry-N1 (i.e., mcherry), 345bp/225bp=7.3, that is, most of the mRNAs were the ones unsuccessfully spliced and containing introns; while in the case of co-transfection with the vector p4-RHO and the vector pMcherry-PRPF31 (i.e., m31), due to the co-expression of the RHO gene and Mcherry-PRPF31, 345bp/225bp=1.9. It could be seen a 4-fold improvement of the splicing ratio. This indicated that the presence of PRPF31 could promote the mRNA splicing of the RHO minigene.

Fig. 4 verified that the vector pMcherry-PRPF31 could successfully express the Mcherry-PRPF31 fusion protein.

### Example 3. Determination of the effects of different NLS sequences on the PRPF31 localization

In order to study whether the NLS would influence the PRPF31 function, the nuclear localization signal in the PRPF31 protein was replaced. That is, the nuclear localization sequence in the PRPF31 protein (the amino acid sequence set forth in SQE ID NO. 15) was replaced by SV40 (the amino acid sequence set forth in SEQ ID NO. 1), A1 (the amino acid sequence set forth in SEQ ID NO. 2), D (the amino acid sequence set forth in SEQ ID NO. 4), M (the amino acid sequence set forth in SEQ ID NO. 5), NP (the amino acid sequence set forth in SEQ ID NO. 6), SRY (the amino acid sequence set forth in SEQ ID NO. 7), or TAT (the amino acid sequence set forth in SEQ ID NO. 3), respectively.

The nucleotide sequences encoding these nuclear localization sequences after replacements were as follows: SV40 (the nucleotide sequence set forth in SEQ ID NO. 8), A1 (the nucleotide sequence set forth in SEQ ID NO. 9), D (the nucleotide sequence set forth in SEQ ID NO. 11), M (the nucleotide sequence set forth in SEQ ID NO. 12), NP (the nucleotide sequence set forth in SEQ ID NO. 13), SRY (the nucleotide sequence set forth in SEQ ID NO. 14), or TAT (the nucleotide sequence set forth in SEQ ID NO. 10).

The nucleic acid molecules encoding the PRPF31 variants after the above replacements were cloned into the vector pMcherry-N1 in accordance with the steps described in Example 1, respectively, to obtain the vector pMcherry-SV40 (abbreviated as SV40), the vector pMcherry-A1 (abbreviated as A1), the vector pMcherry-D (abbreviated as D), the vector pMcherry-M (abbreviated as M), the vector pMcherry-NP (abbreviated as NP), the vector pMcherry-SRY (abbreviated as SRY), and the vector pMcherry-TAT (abbreviated as TAT), respectively.

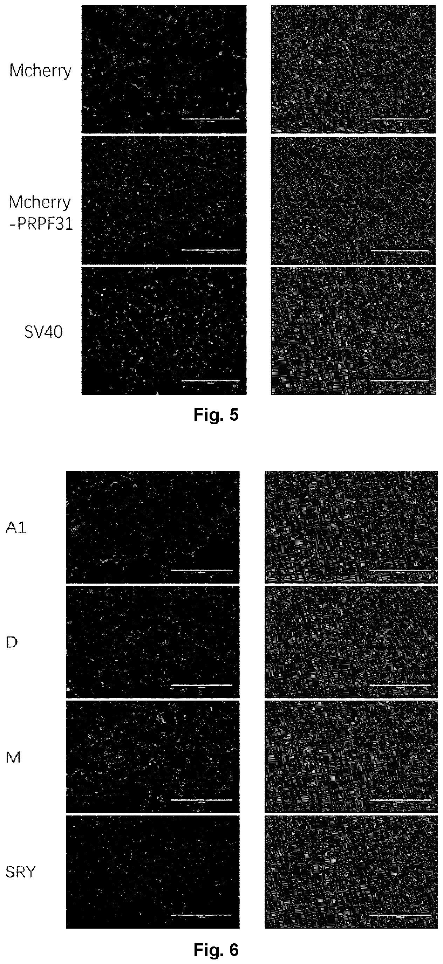

In accordance with the steps described in Example 1, the expression distributions of the nucleic acid molecules contained in the above vectors in the cells were detected. The results were shown in Fig. 5 and Fig. 6, wherein the left columns showed the red fluorescence expressions, and the right columns showed the red fluorescence expressions after merging with bright field.

### Example 4. Determination of the effects of different NLS sequences on the mRNA splicing of the RHO reporter gene

In accordance with the procedures described in Example 2, the cells were co-transfected with the vector p4-RHO containing the RHO minigene reporter gene (SQE ID NO. 33) and each of the vector pMcherry-SV40 (abbreviated as SV40), the vector pMcherry-A1 (abbreviated as A1), the vector pMcherry-D (abbreviated as D), the vector pMcherry-M (abbreviated as M), the vector pMcherry-NP (abbreviated as NP), the vector pMcherry-SRY (abbreviated as SRY), and the vector pMcherry-TAT (abbreviated as TAT) as prepared in Example 3, respectively, or the cells were co-transfected with the vector p4-RHO and each of the control vector pMcherry-N1 (i.e., mcherry) or the vector pMcherry-PRPF31 (i.e., mcherry-PRPF31) as prepared in Example 1, respectively, and the cells without plasmid transfection were used as blank controls.

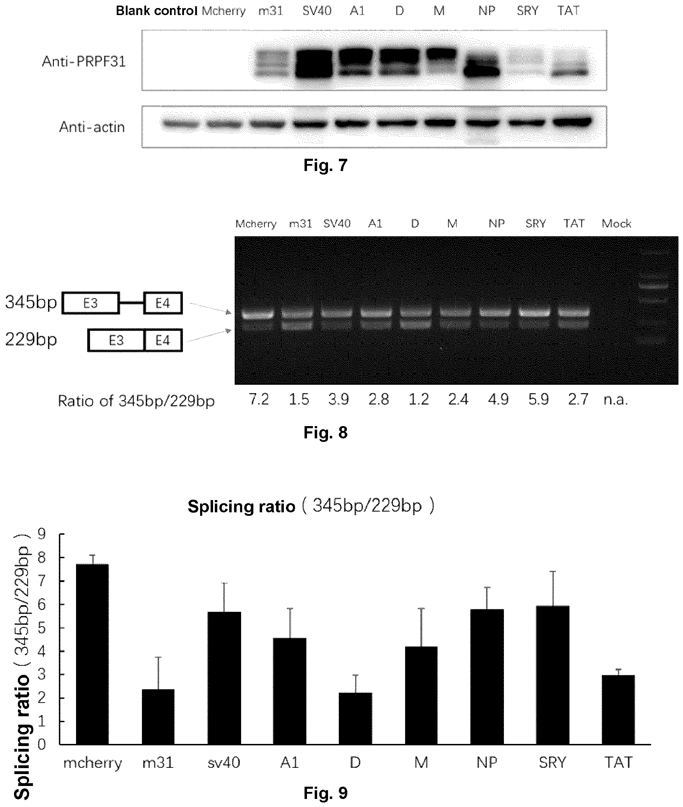

Fig. 7 verified that all of the vector SV40, the vector A1, the vector D, the vector M, the vector NP, the vector SRY, and the vector TAT could successfully express the corresponding Mcherry-PRPF31 fusion protein variants.

Fig. 8 and Fig. 9 showed the effects of the vector SV40, the vector A1, the vector D, the vector M, the vector NP, the vector SRY, and the vector TAT on the mRNA splicing of the RHO reporter gene. The results in Fig. 8 and Fig. 9 demonstrated that the PRPF31 variants with different NLS sequences had different mRNA splicing efficiencies for the RHO reporter gene. Among them, the PRPF31 variants with sequence D or TAT as NLS sequence had a relatively higher mRNA splicing efficiency for the reporter gene, and the PRPF31 variants with sequence D as NLS sequence had a slightly higher mRNA splicing efficiency than PRPF31.

The PRPF31 variants with other NLS sequences had slightly lower splicing efficiencies than PRPF31.

### Example 5. PRPF31 or variants thereof promoted the phagocytosis of the human IPSC-induced RPE cells

Based on the vector pMcherry-PRPF31 as prepared in Example 1, the vector D as prepared in Example 3, and the control vector pMcherry-N1, the nucleic acid molecule encoding Mcherry-PRPF31 (the PRPF31 nucleotide sequence set forth in SEQ ID NO. 25), the nucleic acid molecule encoding Mcherry-PRPF31 variant (sequence D as NLS sequence, and the nucleotide sequence of the variant set forth in SEQ ID NO. 29), and the nucleic acid molecule encoding Mcherry (the nucleotide sequence set forth in SEQ ID NO. 34) were obtained.

The above nucleic acid molecules were cloned into the viral expression vector pFG12 (Addgene, #14884), respectively. Three plasmids were co-transfected into 293T cells for lentivirus package, and the lentiviruses were concentrated and enriched before infecting the human IPSC-induced RPE cells. Eight days after infection, the rat POS coupled with FITC was added to observe the phagocytosis of rat POS by RPE.

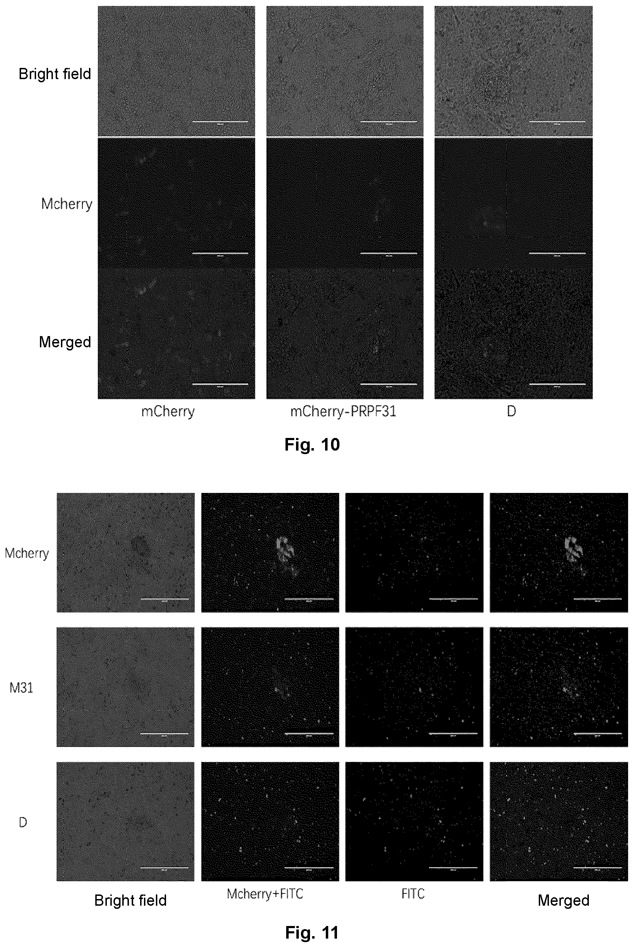

The results were shown in Figs. 10-12. The results in Figs. 10-12 demonstrated that PRPF31 or NLS variants thereof could promote the phagocytosis of the human IPSC-induced RPE cells.

### Example 6. PRPF31 or variants thereof promoted the cilium length of the human IPSC-induced RPE cells

Based on the vector pMcherry-PRPF31 as prepared in Example 1, the vector D as prepared in Example 3, and the control vector pMcherry-N1, the nucleic acid molecule encoding Mcherry-PRPF31 (the PRPF31 nucleotide sequence set forth in SEQ ID NO. 25), the nucleic acid molecule encoding Mcherry-PRPF31 variant (sequence D as NLS sequence, and the nucleotide sequence of the variant set forth in SEQ ID NO. 29), and the nucleic acid molecule encoding Mcherry (the nucleotide sequence set forth in SEQ ID NO. 34) were obtained.

The above nucleic acid molecules were cloned into the viral expression vector pFG12 (Addgene, #14884), respectively. Three plasmids were co-transfected into 293T cells for lentivirus package, and the lentiviruses were concentrated and enriched before infecting the patient IPSC-induced RPE cells.

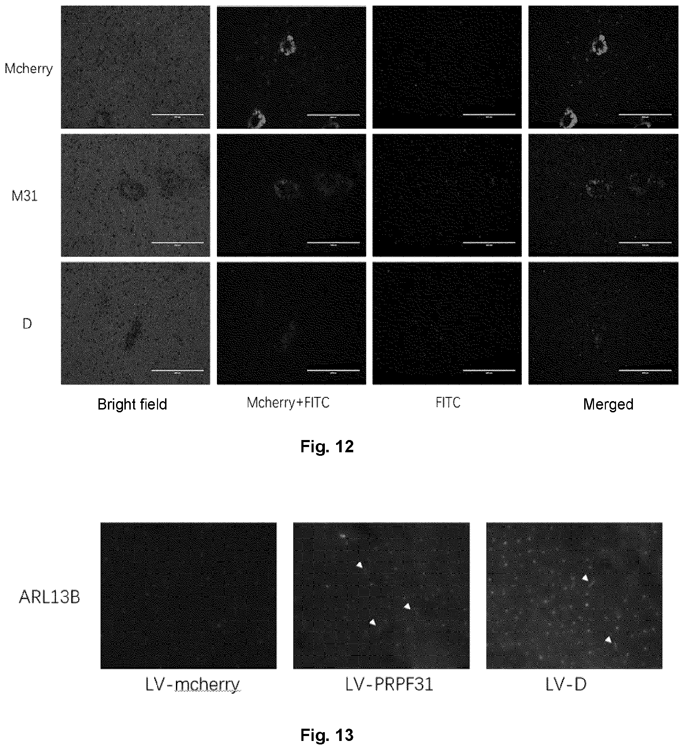

Eight days after infection, the cells were fixed and immunostained by anti-ARL13 antibody (Proteintech). The results in Fig. 13 demonstrated that both PRPF31 and NLS variants thereof could promote the cilium length of the patient IPSC-induced RPE cells.

### Example 7. AAVs expressing PRPF31 or variants thereof promoted the phagocytosis and the cilium length of the human IPSC-induced RPE cells

The CDS of PRPF31 or the CDS portion of variant D was cloned into the pAV-CAG expression vector. Three plasmids were co-transfected into 293T cells for recombinant adeno-associated virus (AAV) package, and the viruses were concentrated and enriched before infecting the patient IPSC-induced RPE cells.

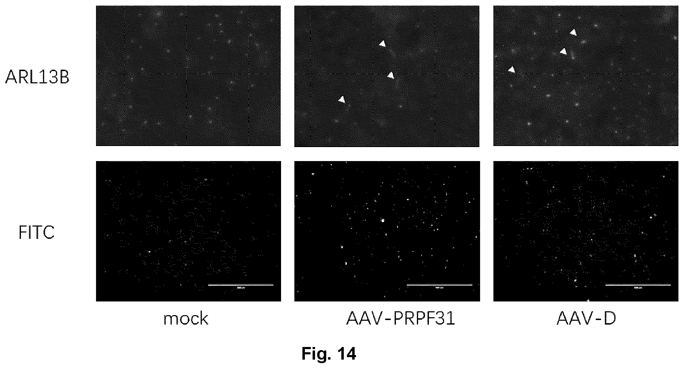

21 days after infection, the rat POS coupled with FITC (homemade) was added to one half wells of AAV-infected iPSC-RPE cells to observe the phagocytosis of rat POS by RPE. The other half wells of AAV-infected iPSC-RPE cells were fixed and immunostained by using anti-ARL13 antibody (Proteintech). The results in Fig. 14 demonstrated that both PRPF31 and variants thereof could promote the phagocytosis of the human IPSC-induced RPE cells as well as the cilium length of the patient IPSC-induced RPE cells.

The foregoing detailed descriptions are provided by way of explanation and illustration, and are not intended to limit the scope of the appended claims. Various modifications to the embodiments presently exemplified in the present application will be apparent to those of ordinary skill in the art and fall into the scope of the appended claims and equivalents thereof.

## Claims

1. A PRPF31 variant comprising an exogenous nuclear localization (NLS) domain or truncated version thereof, wherein the exogenous NLS domain comprises an amino acid sequence set forth in any of SEQ ID NOs. 3 to 4.

2. The PRPF31 variant according to claim 1, which does not comprise the NLS domain of wild-type PRPF31.

3. The PRPF31 variant according to any of claims 1-2, comprising an NOSIC domain.

4. The PRPF31 variant according to any of claims 1-3, comprising an NOP domain.

5. The PRPF31 variant according to any of claims 1-4, which is capable of splicing the mRNA of a gene, wherein the gene comprises RHO gene.

6. The PRPF31 variant according to any of claims 1-5, wherein the PRPF31 variant is derived from human PRPF31.

7. The PRPF31 variant according to any of claims 1-6, comprising an amino acid sequence set forth in any of SEQ ID NOs. 20 to 21.

8. A nucleic acid molecule encoding the PRPF31 variant according to any of claims 1-7.

9. The nucleic acid molecule according to claim 8, comprising a nucleotide sequence set forth in any of SEQ ID NOs. 28 to 29.

10. A vector comprising the nucleic acid molecule according to any of claims 8-9.

11. The vector according to claim 10, comprising a viral vector.

12. The vector according to any of claims 10-11, comprising a lentiviral vector and an AAV vector.

13. The vector according to claim 12, wherein the lentivirus comprises human immunodeficiency virus (HIV), simian immunodeficiency virus (SIV), and non-primate lentiviruses such as feline immunodeficiency virus (FIV), equine infectious anemia virus (EIAV), bovine immunodeficiency virus (BIV), and/or Visna-Maedi virus (VMV).

14. The vector according to any of claims 12-13, wherein the serotype of the AAV vector comprises AAV1, AAV2, AAV5, AAV6, AAV8, AAV9, and/or AAV10.

15. Use of the PRPF31 variant according to any of claims 1-7, the nucleic acid molecule according to any of claims 8-9, and/or the vector according to any of claims 10-14 in the manufacture of a medicament for treating a disease.

16. The use according to claim 15, wherein the disease comprises a disease caused by PRPF31 gene mutation.

17. The use according to any of claims 15-16, wherein the disease comprises retinitis pigmentosa (RP).

18. The use according to any of claims 15-17, wherein the disease comprises autosomal dominant retinitis pigmentosa (ADRP).

19. A method of improving the biological activity of a retinal pigment epithelium (RPE) cell, comprising the step of administrating to the RPE cell the PRPF31 variant according to any of claims 1-7, the nucleic acid molecule according to any of claims 8-9, and/or the vector according to any of claims 10-14.

20. The method according to claim 19, wherein the biological activity comprises the phagocytic activity of the RPE cell, and/or the cilium length of the PRE cell.

21. The method according to any of claims 19-20, wherein the induced RPE cell is derived from a pluripotent stem cell and/or a totipotent stem cell.

22. The method according to any of claims 19-21, wherein the induced RPE cell is derived from a human iPS cell.

23. The method according to any of claims 19-22, wherein the PRPF31 variant does not comprise the NLS domain of wild-type PRPF31.

24. The method according to any of claims 19-23, wherein the PRPF31 variant comprises an amino acid sequence set forth in any of SEQ ID NOs. 20 to 21.

25. The method according to any of claims 19-24, wherein the administrating comprises administrating a vector comprising a nucleic acid molecule encoding the PRPF31 variant.

26. The method according to claim 25, wherein the nucleic acid molecule comprises a nucleotide sequence set forth in any of SEQ ID NOs. 28 to 29.

27. The method according to any of claims 19-26, wherein the vector comprises a viral vector.

28. A method of improving the mRNA splicing efficiency, comprising the step of administrating to a cell containing the mRNA a PRPF31 variant, wherein the PRPF31 variant comprises an exogenous nuclear localization (NLS) domain, and the exogenous NLS domain comprises an amino acid sequence set forth in any of SEQ ID NOs. 3 to 4.

29. The method according to claim 28, wherein the PRPF31 variant does not comprise the NLS domain of wild-type PRPF31.

30. The method according to any of claims 28-29, wherein the PRPF31 variant comprises an amino acid sequence set forth in any of SEQ ID NOs. 20 to 21.

31. The method according to any of claims 28-30, wherein the administrating comprises administrating a vector comprising a nucleic acid molecule encoding the PRPF31 variant.

32. The method according to claim 31, wherein the nucleic acid molecule comprises a nucleotide sequence set forth in any of SEQ ID NOs: 28 to 29.

33. The method according to any of claims 31-32, wherein the vector comprises a viral vector.
